# EUROPÄISCHE PATENTSCHRIFT

(11) **EP 2 280 633 B1**
(45) Veröffentlichungstag und Bekanntmachung des Hinweises auf die Patenterteilung: **19.08.2015**
(21) Anmeldenummer: 09757222.6
(22) Anmeldetag: 28.05.2009
(51) Int. Cl.: A61B 1/12

(54) **VERFAHREN ZUR PRÜFUNG DER DURCHGÄNGIGKEIT EINES ENDOSKOPKANALS UND ENDOSKOPWASCHMASCHINE DAFÜR**
METHOD FOR TESTING THE PATENCY OF AN ENDOSCOPIC CHANNEL, AND ENDOSCOPE WASHING MACHINE FOR SAME
PROCÉDÉ POUR CONTRÔLER LE LIBRE PASSAGE DANS UN CANAL ENDOSCOPIQUE ET APPAREIL DE LAVAGE D ENDOSCOPE À CET EFFET

(30) Priorität: 03.06.2008 DE 102008026445
(43) Veröffentlichungstag der Anmeldung: 09.02.2011
(73) Patentinhaber: OLYMPUS WINTER & IBE GMBH, 22045 Hamburg (DE)
(72) Erfinder: WALDMANN, Jens, 22393 Hamburg (DE); ESCHBORN, Sascha, 22926 Ahrensburg (DE)
(74) Vertreter: Hausfeld, Norbert
(86) Internationale Anmeldenummer: PCT/EP2009/003819
(87) Internationale Veröffentlichungsnummer: WO 2009/146839

(56) Entgegenhaltungen:
- EP-A- 0 709 056
- EP-A- 0 711 529
- EP-A- 1 779 769
- US-A1- 2007 100 204

## Beschreibung

Die Erfindung betrifft ein Verfahren nach dem Oberbegriff des Anspruches 1 sowie eine Endoskopwaschmaschine nach dem Oberbegriff des Anspruches 3.

Endoskope, insbesondere flexible Endoskope, weisen lange und enge Kanäle auf, die z. B. der Flüssigkeits- oder Gasführung dienen. Bei der maschinellen Reinigung und Desinfektion eines Endoskops in einer Endoskopwaschmaschine müssen auch die Kanäle durchspült werden. Zur Gewährleistung eines ordnungsgemäßen Reinigungs- und Desinfektionsergebnisses ist eine vorherige Prüfung der Endoskopkanäle auf Durchgängigkeit erforderlich. Endoskopwaschmaschinen sind mit entsprechenden Einrichtungen ausgerüstet.

Eine gattungsgemäße Endoskopwaschmaschine ist aus der EP 0 709 056 A1 bekannt. Bei ihr wird zur Durchgängigkeitsprüfung der zu prüfende Kanal des Endoskops unter Druck gesetzt und sodann abgesperrt und der anschließende Druckverlust aufgezeichnet. Hierzu sind sehr genaue Druckmesser sowie ein aufwändiges Messverfahren erforderlich und es ist schwierig zwischen unterschiedlichen Stadien der Durchgängigkeit zu unterscheiden.

Die Aufgabe der vorliegenden Erfindung besteht darin, eine Durchgängigkeitsprüfung zu schaffen, die auf einfache Weise klare Ergebnisse schafft.

Diese Aufgabe wird mit den Merkmalen des Anspruches 1 sowie des Anspruches 3 gelöst.

Erfindungsgemäß wird der zu prüfende Kanal mit einer Folge von Druckimpulsen beaufschlagt. Ein mit einer Impulsfolge angesteuertes Schaltventil kann diese auf einfache Weise erzeugen. Zwischen dem Ventil und dem Eingang des Kanals wird der Druck gemessen. Es ergeben sich die gemessenen Druckimpulse, die sich je nach Durchgängigkeit des Kanals in charakteristischer Weise unterscheiden. Ist der Kanal normal durchgängig, so ergeben sich die Impulse mit dem vollen Druckhub im wesentlichen zwischen 0 und dem Pumpendruck. Ist der Kanal blockiert, so ergeben sich dieselben Impulse, jedoch mit stark verringerter Impulshöhe und zwar auf absolut angehobenem Druckniveau im Bereich des Pumpendruckes. Ist der Kanal gar nicht angeschlossen (ein unabdingbar wichtiger Prüfpunkt) dann liegen die Impulse mit geringem Hub auf niedrigem absoluten Druckniveau im Bereich von 0. Diese drei unterschiedlichen Ergebnisse lassen sich sehr einfach und klar unterscheiden und führen mit einfachen Mitteln und sehr schnell zu einer klaren Aussage über die Durchgängigkeit des Kanals. Vorteilhaft gemäß Anspruch 2 werden nur zwei Werte bestimmt, nämlich die relative Höhe der Impulse und deren absolute Höhenlage. Dadurch vereinfacht sich die Messung auf das Notwendige.

Eine Endoskopwaschmaschine zur Durchführung des erfindungsgemäßen Verfahrens ist in Anspruch 3 angegeben. Eine vorteilhafte Ausgestaltung ist in Anspruch 4 angegeben. Die elastische Volumenvergrößerbarkeit der Verbindungsleitung sorgt für eine gewisse Druckspeicherung bei geschlossenem Schaltventil, so dass im Falle des blockierten Kanals der Druck auch bei geschlossenem Ventil hoch bleibt.

In der Zeichnung ist die Erfindung beispielsweise und schematisch dargestellt. Es zeigen:
- Fig. 1: ein Blockschaltbild einer Einrichtung zur Durchgängigkeitsprüfung und
- Fig. 2 - 4: Impulsdiagramme für unterschiedliche Testergebnisse.

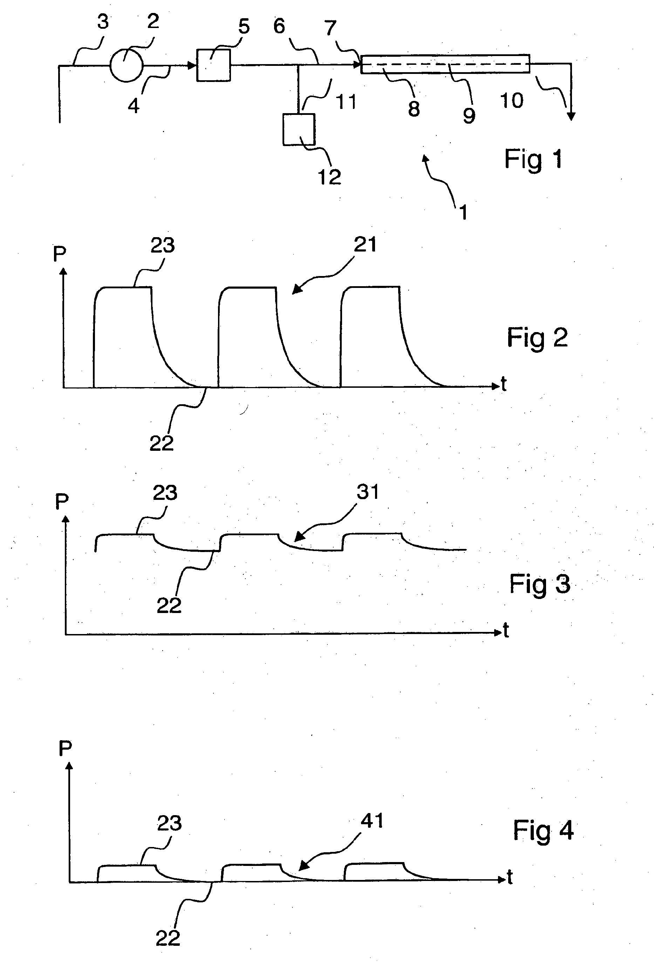

Fig. 1 zeigt einen Teil einer im Weiteren nicht näher dargestellten Endoskopwaschmaschine, nämlich eine Einrichtung 1 zur Durchgängigkeitsprüfung. Diese weist eine Pumpe 2 auf, die mit einer Ansaugleitung 3 Spülflüssigkeit ansaugt und über eine Druckleitung 4 an ein Schaltventil 5 gibt, welches derart gesteuert ist, dass es in einer Impulsfolge öffnet und schließt. Eine Verbindungsleitung 6 führt von dem Schaltventil 5 zum Eingang 7 eines in einem Endoskop 8 angeordneten, gestrichelt dargestellten Kanals 9, von dessen Ausgang die Flüssigkeit über eine Ablassleitung 10 abfließt.

An der Verbindungsleitung 6 ist über eine Zweitleitung 11 ein Druckmesser 12 angeschlossen.

Das Schaltventil 5 wird in Steuerung durch eine Impulsfolge geöffnet und geschlossen und erzeugt somit auf der Verbindungsleitung 6 Impulse, wie sie in den Figuren 2 - 4 dargestellt sind und zwar jeweils mit dem Druck P aufgetragen über der Zeit t.

Figur 2 zeigt die von dem Druckmesser 12 auf der Verbindungsleitung 6 gemessenen Impulsfolge 21 der Druckimpulse für den Fall eines normal durchgängigen Kanals 9. Bei geöffnetem Schaltventil 5 steigt der Druck bis auf den dem Pumpendruck entsprechenden Maximalwert 23 an und fällt bei geschlossenem Ventil auf den Minimalwert 22, also im Wesentlichen bis auf 0 ab, da die Flüssigkeit rasch durch den Kanal 9 abströmen kann. Dieses Ergebnis ist also charakterisiert durch den großen Impulshub.

Figur 3 zeigt die gemessene Impulsfolge 31 für den Fall eines blockierten Kanals 9. Wenn das Schaltventil 5 geschlossen ist, kann die Flüssigkeit nicht, oder nur in sehr geringem Maße durch den blockierten Kanal 9 ablaufen. Der Druck bleibt also auch in den Impulspausen also bei den Minimalwerten 22 auf hohem Druckniveau, wie dies die Figur 3 zeigt. Es werden Impulse mit niedrigem Hub auf einem absolut hohen Druckniveau gemessen.

Figur 4 zeigt die die gemessene Impulsfolge 41 im Falle, dass die Verbindungsleitung 6 nicht ordnungsgemäß an den Eingang 7 des Kanals 8 angeschlossen ist. Flüssigkeit kann ohne jeden Gegendruck aus der Verbindungsleitung 6 entweichen. Es kann sich in dieser nur ein sehr geringer Druck aufbauen, wenn das Schaltventil 5 öffnet. Es ergeben sich also, wie Figur 4 zeigt, Impulse mit niedrigem Hub auf niedrigem Druckniveau.

Die Ergebnisse gemäß den Figuren 2 - 4 lassen sich auf den ersten Blick auch von ungeübtem Personal und von einfachen Maschinen verwechslungssicher unterscheiden. Es kann also mit einer sehr einfachen Maschine und in kurzer Messzeit zwischen den wesentlichen Durchgängigkeitssituationen unterschieden werden, die bei einem Endoskopkanal vorliegen können, nämlich normal durchgängiger und korrekt angeschlossener Kanal mit dem Ergebnis der Figur 2, blockierter Kanal mit dem Ergebnis der Figur 3 und nicht angeschlossener Kanal mit dem Ergebnis der Figur 4.

Die Frequenz der bei der dargestellten Einrichtung 1 zum Steuern des Schaltventils 5 verwendeten Impulse kann in der Größenordnung 1 Hz liegen.

Wie die Figuren 2 - 4 zeigen, sind die sich ergebenden Druckimpulse in den Flankenbereichen etwas verrundet, was die Ergebnisse aber in keiner Weise stört, bei denen es lediglich auf die Bestimmung der charakteristischen Unterschiede zwischen den Figuren 2 bis 4 ankommt. Dafür spielt die genaue Druckimpulsform keine Rolle. Es müssen lediglich die jeweiligen Maximalwerte 23 und Minimalwerte 22 bestimmt werden.

Die Verbindungsleitung 6 sollte eine gewisse Volumenelastizität aufweisen. Sie kann z. B. als elastische Schlauchleitung ausgebildet sein oder an ein elastisches Ausdehnungsgefäß oder dergleichen angeschlossen sein. Damit wird eine gewisse Druckspeicherung gewährleistet, die bei geschlossenem Schaltventil 5 das Hochhalten des Druckes gemäß Figur 3 ermöglicht.

## Patentansprüche

1. Verfahren zur Prüfung der Durchgängigkeit eines Endoskopkanals (9) in einer Endoskopwaschmaschine, wobei der Eingang (7) des Kanals (9) unter Druck gesetzt und dann abgesperrt wird, worauf die Druckänderung am Eingang (7) gemessen wird, **dadurch gekennzeichnet, dass** der Kanal (9) mit einer Folge von Druckimpulsen (21, 31, 41) beaufschlagt wird und deren Maximal- und Minimalwerte (23, 22) bestimmt werden.

2. Verfahren nach Anspruch 1, **dadurch gekennzeichnet, dass** die relative Höhe der Impulse und ihre absolute Höhenlage gemessen wird.

3. Endoskopwaschmaschine mit einer Einrichtung (1) zur Prüfung der Durchgängigkeit eines Endoskopkanals (9), wobei diese Einrichtung eine Pumpe (2), ein Schaltventil (5) und einen Druckmesser (12) aufweist, wobei das von der Pumpe versorgte und mit einer Pulsfolge gesteuerte Schaltventil und der Druckmesser an einer an den Kanal anschließbaren Verbindungsleistung (6) angeschlossen sind, **dadurch gekennzeichnet, dass** die Endoskop-Waschmaschine zur Durchführung des Verfahrens nach einem der vorhergehenden Ansprüche ausgebildet ist, nämlich zur Beaufschlagung des Kanals mit einer Folge von Druckimpulsen und zur Bestimmung von deren Maximal- und Minimalwerten.

4. Endoskopwaschmaschine Anspruch 3, **dadurch gekennzeichnet, dass** die Verbindungsleitung (6) elastisch volumenvergrößerbar ausgebildet ist.

## Claims

1. A method for checking the patency of an endoscope channel (9) in an endoscope washing machine, wherein the inlet (7) of the channel (9) is put under pressure and then closed, whereupon the change in pressure is measured at the inlet (7), **characterised in that**
- a series of pressure pulses (21, 31, 41) is applied to the channel (9) and the maximum and minimum values (23, 22) thereof are determined.

2. The method according to Claim 1,
**characterised in that**
- the relative height of the pulses and their absolute height position are measured.

3. An endoscope washing machine having a device (1) for checking the patency of an endoscope channel (9), wherein this device comprises a pump (2), a switching valve (5) and a pressure gauge (12), wherein the switching valve that is supplied by the pump and controlled by means of a series of pulses and the pressure gauge are connected to a connecting line (6) that can be connected to the channel, **characterised in that**
- the endoscope washing machine is formed to apply the method according to any one of the preceding claims, i.e., to apply a series of pressure pulses to the channel and to determine the maximum and minimum values thereof.

4. The endoscope washing machine according to Claim 3,
**characterised in that**
- the connecting line (6) is formed such that its volume can be increased in an elastic manner.

## Revendications

1. Procédé de contrôle du libre passage d'un canal d'endoscope (9) dans un appareil de lavage d'endoscopes, l'entrée (7) du canal (9) étant mise sous pression puis obturée, après quoi la modification de pression est mesurée au niveau de l'entrée (7), **caractérisé en ce que** le canal (9) est soumis à une série d'impulsions de pression (21, 31, 41) et **en ce que** les valeurs maximales et minimales (23,22) en sont déterminées.

2. Procédé selon la revendication 1, **caractérisé en ce que** l'amplitude relative des impulsions et leur niveau absolu sont mesurés.

3. Appareil de lavage d'endoscopes doté d'un dispositif (1) de contrôle du libre passage d'un canal d'endoscope (9), ce dispositif présentant une pompe (2), une soupape de commande (5) et un manomètre (12), la soupape de commande alimentée par la pompe et commandée par une série d'impulsions et le manomètre étant branchés sur une conduite de connexion (6) pouvant être branchée sur le canal, **caractérisé en ce que** l'appareil de lavage d'endoscopes est conçu pour la mise en oeuvre du procédé selon l'une des revendications précédentes, à savoir soumettre le canal à une série d'impulsions de pression et en déterminer les valeurs maximales et minimales.

4. Appareil de lavage d'endoscopes selon la revendication 3, **caractérisé en ce que** la conduite de raccordement (6) est réalisée de façon à en permettre l'augmentation élastique de volume.
